# EUROPEAN PATENT SPECIFICATION

(11) **EP 4 278 997 B1**
(45) Date of publication and mention of the grant of the patent: **29.10.2025**
(21) Application number: 23174195.0
(22) Date of filing: 18.05.2023
(51) Int. Cl.: A61B 17/58, A61B 17/72

(54) **DEPLOYABLE BONE ANCHORS FOR ORTHOPEDIC IMPLANTS**
ENTFALTBARE KNOCHENANKER FÜR ORTHOPÄDISCHE IMPLANTATE
ANCRAGES OSSEUX DÉPLOYABLES POUR IMPLANTS ORTHOPÉDIQUES

(30) Priority: 19.05.2022 US 202263343768 P
(43) Date of publication of application: 22.11.2023
(73) Proprietor: Zimmer, Inc., Warsaw, IN 46580 (US)
(72) Inventor: DYE, Donald W., Warsaw, 46582 (US)
(74) Representative: Allen, Caroline Margaret

(56) References cited:
- EP-A2- 1 931 266
- US-A1- 2009 292 313
- US-A1- 2011 319 946
- US-A1- 2012 271 363
- US-A1- 2017 303 977

## Description

### CLAIM OF PRIORITY

This application claims the benefit of U.S. Provisional Patent Application Serial No. 63/343,768, filed on May 19, 2022.

### TECHNICAL FIELD

The present disclosure is directed to systems, devices and methods for affixing orthopedic implants within a joint. More particularly, the present disclosure is directed to bone anchors that can be inserted into cancellous bone matter for attaching an orthopedic implant to a bone.

### BACKGROUND

Orthopedic implants are commonly used to replace some or all of a joint of a patient in order to restore use of the joint, or to increase use of the joint, following deterioration due to aging or illness, or injury due to trauma. Orthopedic implants can be used to restore hip joints, shoulder joints, ankle joints, knee joints and others. For example, in a shoulder replacement, or shoulder arthroplasty procedure, a humeral component is used to replace a portion of the humerus bone of the patient, including the humeral head. The humeral component can typically include a stem that can be inserted into a canal prepared in the cancellous bone of the humerus. The stem can be inserted via a press-fitting action and held in place by frictional engagement. Additionally, bone cement can be used to further prevent displacement of the stem. The frictional engagement and bone cement can hold the stem in place until boney ingrowth of the surrounding tissue into porous areas in the stem occurs. The humeral stem can include a neck portion, or another feature adapted to receive a prosthetic humeral head. The prosthetic humeral head can be received within the anatomic glenoid or a prosthetic glenoid component, such as a cup implanted in the glenoid of the scapula of the patient. Similar devices and procedures can be used in other joints, including hip joints and knee joints.

Pat. No. US 7,488,329 to Thelen et al., titled "Method and Apparatus for Reducing Femoral Fractures" and Pat No. US 8,506,638 to Vanasse et al., titled "Shoulder Prosthesis" describe various examples of orthopedic implants. Patent document US 2009/292313 A1 discloses a deployable bone anchor for an orthopaedic implant, the deployable bone anchor comprising: an implantable body comprising:a first end comprising:a first attachment member to facilitate coupling of a prosthetic component to the implantable body; and a second attachment member to facilitate coupling of a deployment device to the implantable body; a second end for insertion into a bone; and wings connecting the first end and the second end, the wings comprising: a living hinge portion configured to allow the wings to flex when the second attachment member and the second end of the implantable body are pulled toward each other by the deployment device when the deployment device is attached to the implantable body; wherein the wings are configured to deploy from an extended configuration where the wings are axially extending to a collapsed configuration where the wings are bowed radially outward; and wherein the living hinge portion is configured to induce curvature to the wings in the collapsed configuration.

### OVERVIEW

The present inventor has recognized, among other things, that problems to be solved in conventional arthroplasty procedures involve the removal of cancellous bone from the bone of the patient that can potentially hold bone anchors in place. Many bone anchors are designed to displace cancellous bone via a press-fit insertion process in order to provide a tight fit between the bone and the bone anchor. Press-fitting of the bone anchor is typically accomplished by pushing the bone anchor straight into the cancellous bone, thereby producing a tunnel or canal in which the bone anchor resides. The tunnel or canal can disrupt healthy bone matter in the bone, potentially prolonging the healing process. There also remains the potential for the bone anchor to migrate backwards out of the tunnel or canal, particularly if the bone cement or boney ingrowth does not provide suitable fixation. Furthermore, conventional bone anchors typically provide a one-size-fits-all approach where the cancellous bone is impacted the same for each patient regardless of the specific condition of the cancellous bone of the patient. This can sometimes lead to the undesirable removal of healthy cancellous bone.

The present inventor has recognized that it is desirable to preserve as much bone as possible when implanting a bone anchor for an orthopedic implant. In particular, it is desirable to preserve cancellous bone that attaches to the bone anchor to hold the orthopedic implant in place. The present subject matter can provide solutions to these and other problems, such as by providing bone anchors that can be deployed to change shape. The deployed bone anchors can, for example, be enlarged in cross-sectional area after being inserted into the cancellous bone. The cross-sectional area can be enlarged in various axial and radial positions to engage cancellous bone to prevent backtracking and dislodgment of the bone anchor. Locations for the enlargement can be selected based on condition of cancellous bone in a particular patient or the shape of the bone.

The present invention provides a deployable bone anchor for an orthopedic implant, the deployable bone anchor comprising an implantable body comprising a first end comprising a first attachment member to facilitate coupling of a prosthetic component to the implantable body and a second attachment member to facilitate coupling of a deployment device to the implantable body; a second end for insertion into a bone, the second end comprising a third attachment member to facilitate coupling to the deployment device; and a sidewall connecting the first end and the second end, the sidewall comprising a living hinge portion configured to allow the sidewall to flex when the second attachment member and the third attachment member are pulled toward each other by the deployment device when the deployment device is attached to the implantable body; wherein the sidewall is configured to deploy from an extended configuration where the sidewall is axially extending to a collapsed configuration where the sidewall is bowed radially outward; and wherein the living hinge portion is configured to induce curvature to the sidewall in the collapsed configuration.

Further described herein is a system for attaching an orthopedic implant to bone can comprise a deployment device and a deployable bone anchor. The deployment device can comprise a sleeve extending along an axis and a drive shaft extending through the sleeve. The deployable bone anchor can comprise a first end comprising a socket for receiving a mating feature of a prosthetic component and a coupler for attaching to the sleeve along the axis, a second end for insertion into a bone, the second end comprising a bore for receiving the drive shaft, and a sidewall connecting the first end and the second end, the sidewall comprising a plurality of axially extending panels and a plurality of living hinges to allow the plurality of axially extending panels to rotate radially relative to the axis.

Methods are described herein but the methods are not claimed. A method of implanting a prosthetic component in a bone can comprise forming a bore in a resected surface of a bone, inserting a distal end of a deployable bone anchor into the bore, attaching a deployment device to a proximal end of the deployable bone anchor, radially expanding the deployable bone anchor with the deployment device, detaching the deployment device, and attaching a prosthetic component to the deployable bone anchor.

### BRIEF DESCRIPTION OF THE DRAWINGS

FIG. 1 is a partial cross-sectional view of a prosthetic humeral head implant having a stem implanted into a humeral bone.
FIG. 2A is a diagrammatic side view of a deployable bone anchor of the present disclosure disposed within a humeral head in an unexpanded state.
FIG. 2B is a diagrammatic side view of the deployable bone anchor of FIG. 2A in an expanded state within the humeral bone and attached to a prosthetic humeral head.
FIG. 2C is a cross-sectional view of the expanded deployable bone anchor of FIG. 2B showing the relative sizes of the unexpanded state and the expanded state.
FIG. 3 is a side view of a deployable bone anchor of the present disclosure comprising a sidewall having living hinges.
FIG. 4 is a cross-sectional view of the deployable bone anchor of FIG. 3 showing a prosthetic component attachment member and a deployment device attachment member.
FIG. 5 is a side view of the deployable bone anchor of FIG. 3 with the addition of a plurality of porous structures on the sidewall.
FIGS. 6A, 6B and 6C are side views of deployable bone anchors of the present disclosure in top-heavy, symmetric and bottom-heavy deployment configurations.
FIG. 7 is a side cross-sectional view of a flexible sidewall of a deployable bone anchor of the present disclosure comprising four moveable panels and five living hinges.
FIGS. 8A and 8B are diagrammatic views of an asymmetric deployable bone anchor having a first sidewall panel that radially expands and a second sidewall panel that remains flat upon deployment.
FIGS. 9A and 9B are diagrammatic views of an asymmetric deployable bone anchor having a first sidewall panel that radially expands more than a second sidewall panel.
FIG. 10A is a diagrammatic side view of a deployable bone anchor of the present disclosure comprising variable width sidewall panels and living hinges that can induce curvature of the bone anchor in a deployed state.
FIG. 10B is a diagrammatic side view of the deployable bone anchor of FIG. 10A in the deployed state such that a center line of the bone anchor is curved.
FIG. 11A is a diagrammatic side view of an implanted bone anchor of the present disclosure being separated from bone matter with tools.
FIG. 11B is a perspective view of a distal end of an osteotome gouge that can be used as a tool to separate a deployed bone anchor of the present disclosure from bone matter.
FIG. 12 is a side cross-sectional view of a deployable bone anchor of the present disclosure having features for engaging a deployment device.
FIG. 13 is a side view of the deployable bone anchor of FIG. 12 in an expanded state.
FIG. 14 is a side cross-sectional view of another example of a deployable bone anchor configured to engage a deployment device.
FIG. 15 is a partial cross-sectional view of a deployment device of the present disclosure that can be used to deploy and retrieve deployable bone anchors of the present disclosure.
FIGS. 16A and 16B are block diagrams illustrating steps of methods for implanting and removing deployable bone anchors of the present disclosure using deployment devices.
FIG. 17A is a schematic view of a deployable trauma rod of the present disclosure including a plurality of radially expandable zones.
FIG. 17B is a schematic view of the deployable trauma rod of FIG. 17A in a deployed state such that the radially expandable zones are radially enlarged.
FIG. 18A is a schematic view of a sidewall of a deployable bone anchor of the present disclosure including a plurality of living hinges having hard stops integrated therein.
FIG. 18B is a schematic view of the deployable bone anchor of FIG. 18A in a deployed state such that the integrated hard stops are engaged.

### DETAILED DESCRIPTION

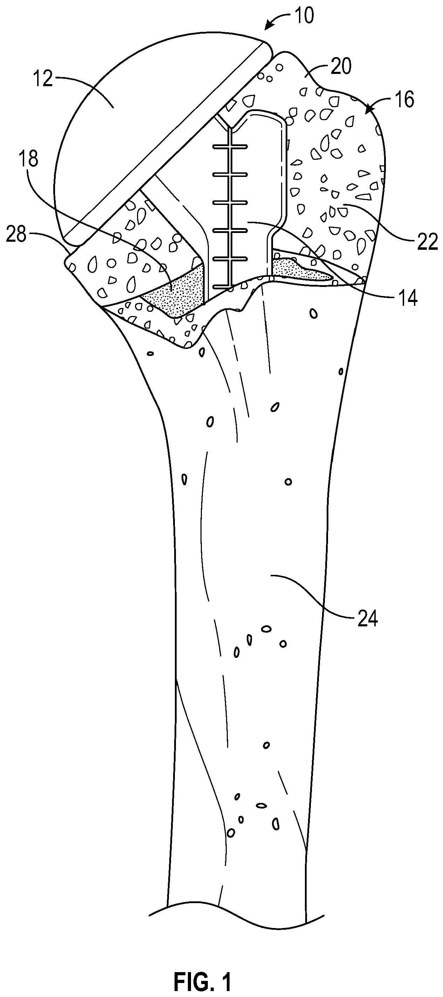

FIG. 1 is a partial cross-sectional view of humeral head prosthesis 10 having head 12 and stem 14. Humeral head prosthesis 10 can be implanted into humerus 16. A portion of humerus 16 is cut-away in FIG. 1 to show stem 14 and cancellous bone 18. In examples, humeral head prosthesis 10 can comprise the prosthetic humeral head system described in Pat. No. US 10,925,738, titled "Adjustable Orthopedic Connections" to Nathan A. Winslow et al..

Humerus 16 can comprise humeral head 20, tubercle area 22 and diaphysis region 24. Humerus 16 can have a hard exterior formed of cortical bone and a softer interior formed of cancellous bone 18. Humeral head 20 can be resected to form cut surface 28 that can expose cancellous bone 18. An intramedullary canal (not visible) can be located in cancellous bone 18 and can extend axially along an interior of humerus 16. Humeral head prosthesis 10 can be attached to humerus 16 via insertion of stem 14 into the intramedullary canal in cancellous bone 18 until head 12 contacts or is in close proximity to cut surface 28. Head 12 and stem 14 can be fabricated of typical materials for prosthetic implants, such as titanium or stainless steel. Such materials can be hard to reduce wear and prevent damage or corrosion.

To implant humeral head prosthesis 10, the intramedullary canal of cancellous bone 18 can be reamed to produce a cavity to receive stem 14. The cavity can be produced to be slightly smaller than stem 14 in order to obtain a tight fit so that humeral head prosthesis 10 is not loose and likely to shift position. Thus, in order to implant and fully seat humeral head prosthesis 10, stem 14 can be pushed into cancellous bone 18 in a linear manner. The press-fitting of stem 14 into the cancellous bone, bone cement and growth of bone material into surface features of stem 14 can affix humeral head prosthesis 10 to humerus 16. However, it can take time for all of these features to produce beneficial results and the performance of any of these attachment mechanisms can become weakened over time. Furthermore, in conventional implants, the shape of stem 14 does not take into account where weak and strong cancellous bone matter is located for a particular patient. Thus, healthy bone and areas of bone that have lower density than healthy bone interact with stem 14 in the same manner.

With the present disclosure, deployable bone anchors can be used to provide immediate fixation of the implanted prosthesis to prevent backward migration from, as well as rotation in, cancellous bone 18. The deployable bone anchors can be radially expanded at one or more axial positions using a deployment device to position portions of the bone anchor in bone matter outside of the tunnel or canal into which the bone anchor is press-fit. As such, bone matter can be positioned axially between the expanded bone anchor and the outside of the bone to impede backward migration of the bone anchor. Additionally, the expanded portions of the bone anchors can be radially variable, e.g., extend over less than three-hundred-sixty-degrees of the perimeter of the bone anchor, to prevent rotation of the bone anchor within the tunnel or canal. Furthermore, the expansion of the bone anchors can be selectively placed to displace unhealthy bone and engage healthy bone to limit disruption or removal of healthy bone. In additional examples of the present disclosure, expansion of the deployable bone anchors can be induced and reversed using the deployment device so that removal of the bone anchor at a later time for a revision procedure, if needed, does not further disrupt or remove cancellous bone matter.

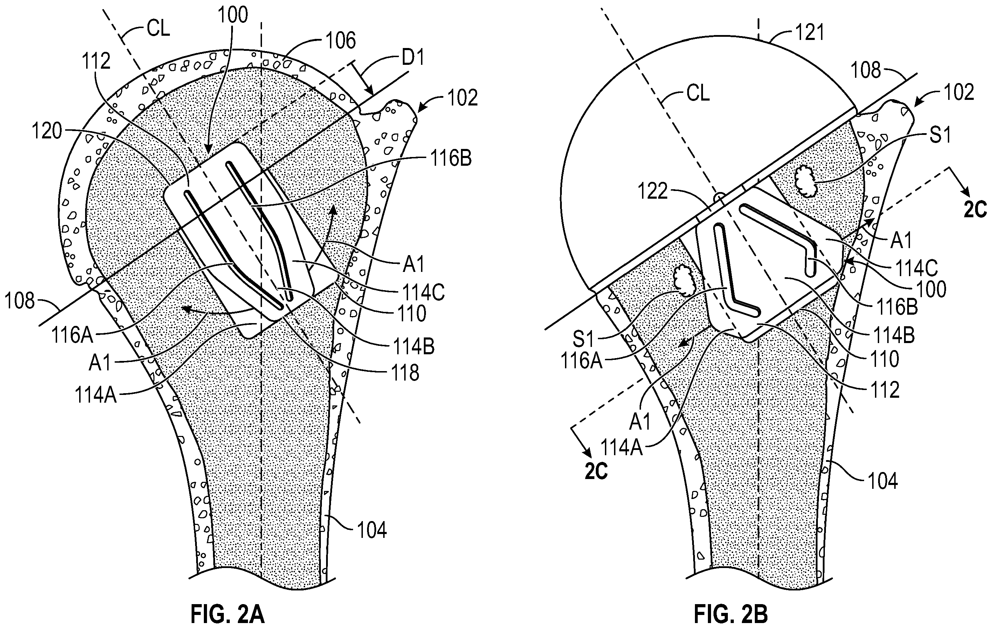

FIG. 2A is a diagrammatic side view of deployable bone anchor 100 of the present disclosure disposed within humerus 102. Humerus 102 can comprise diaphysis region 104 and humeral head 106. Humeral head 106 can be resected along plane 108 to expose a surface of humerus 102 into which bore 110 can be formed. Deployable bone anchor 100 is shown within bore 110 with humeral head 106 intact for illustrative purposes. Deployable bone anchor 100 can comprise body 112 having a sidewall with flexible panels 114A, 114B and 114C that can be formed by slits 116A and 116B. Deployable bone anchor 100 can include additional flexible panels and slits distributed around the perimeter of body 112 relative to center line CL.

Distal end 118 of deployable bone anchor 100 can be inserted into bore 110 such that proximal end 120 extends beyond plane 108 distance D1. Bore 110 and deployable bone anchor 100 can extend axially along center line CL. As discussed in greater detail herein, a deployment device can be attached to proximal end 120 of deployable bone anchor 100 to cause radial expansion of flexible panels 114A - 114C, as indicated by arrows A1. Deployment of deployable bone anchor 100 can simultaneously retract proximal end 120 an amount equal to or greater than distance D1 to bring proximal end 120 flush with plane 108 or into bore 110 and expand the outer perimeter of deployable bone anchor to position portions of deployable bone anchor 100 radially outside of bore 110 within cancellous bone matter.

FIG. 2B is a diagrammatic side view of deployable bone anchor 100 of FIG. 2A in an expanded state within bore 110 of humerus 102 and having prosthetic humeral head 121. Prosthetic humeral head 121 can be attached to deployable bone anchor 100 using coupler 122. In examples, coupler 122 can comprise a projection that can be seated in a socket within proximal end 120 of deployable bone anchor. In examples, prosthetic humeral head 121 and deployable bone anchor 100 can be connected via a Morse taper system. In FIG. 2B. proximal end 120 of bone anchor 100 is retracted into bore 110. Flexible panels 114A - 114C can be flexed to as to bow radially outward. As such, sections S1 of bone matter can be positioned axially between deployed portions of flexible panels 114A, 114B and 114C and plane 108, thereby preventing deployable bone anchor 100 from migrating axially out of bore 110. Flexible panels 114A, 114B and 114C can additionally inhibit rotation about center line CL, such as by the edges of flexible panels 114A - 114C at slits 116A and 116B engaging cancellous bone.

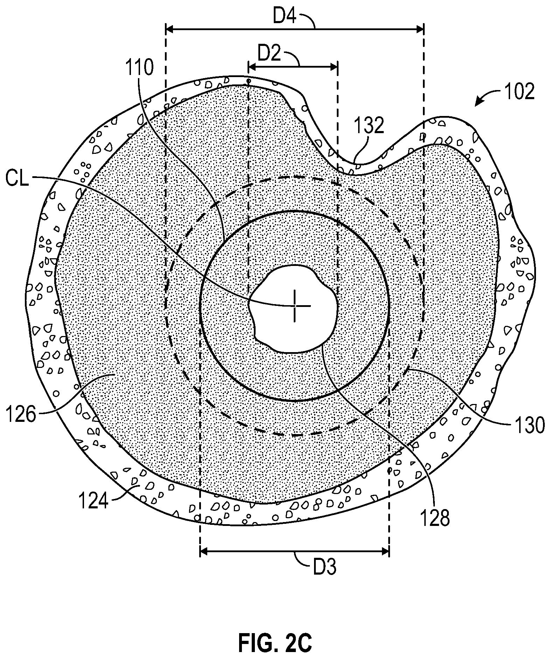

FIG. 2C is a cross-sectional view of expanded deployable bone anchor 100 of FIG. 2B showing the relative sizes of the unexpanded state and the expanded state. Bone anchor 100 is positioned within bore 110 in humerus 102. Humerus 102 can comprise outer cortical bone 124 and inner cancellous bone 126. Intramedullary canal 128 can be located in cancellous bone 126. Bore 110 can be formed in cancellous bone 126 concentric with intramedullary canal 128. Flexible panels 114A, 114B and 114C can be deployed so that body 112 forms outer perimeter 130. Intramedullary canal 128 can have diameter D2, bore 110 can have diameter D3 and perimeter 130 can have diameter D4. The diameter of deployable bone anchor 100 in the unexpanded state can be approximately equal to diameter D3. Additionally, the diameter of distal end 118 and proximal end 120 (FIG. 2B) can be approximately equal to diameter D3. As such, deployable bone anchor 100 can be press-fit into intramedullary canal 128, whether reamed or un-reamed, to form frictional engagement between body 112 and cancellous bone 126. Flexible panels 114A, 114B and 114C can be expanded to be underneath cancellous bone 126 (relative to the view of FIG. 2C) to prevent deployable bone anchor 100 from moving out of bore 110 (up out of the plane of FIG. 2C). As discussed herein, flexible panels 114A, 114B and 114C can be configured to expand different radial distances from center line CL to provide perimeter 130 with a non-uniform shape. Non-uniformity of perimeter 130 can conform deployable bone anchor 100 more closely to the shape of a bone, e.g., to avoid cortical bone, or to interact advantageously with cancellous bone 126, e.g., by displacing unhealthy bone and engaging healthy bone. In examples, perimeter 130 can be configured to be shorter near intertubercular groove 132 or sulcus, between the greater tubercle and the lesser tubercle.

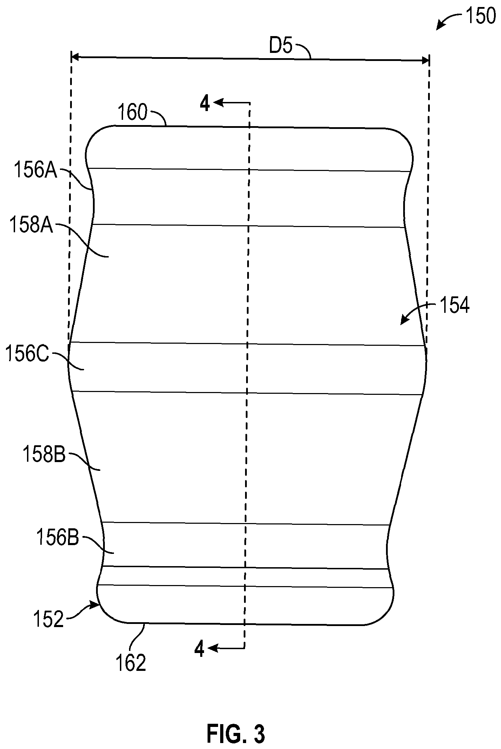

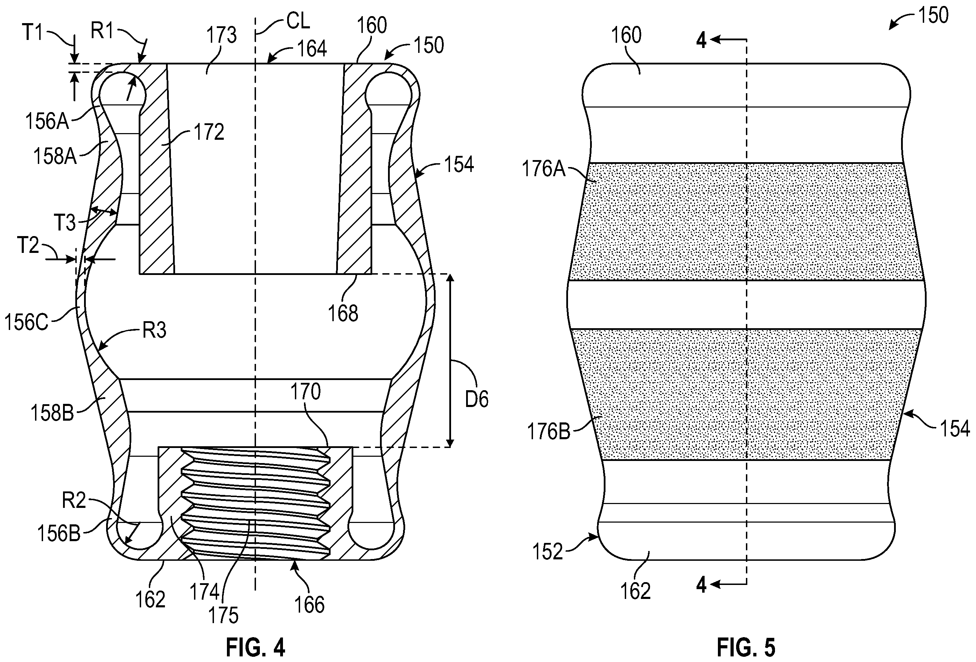

FIG. 3 is a side view of deployable bone anchor 150 of the present disclosure comprising body 152 having sidewall 154 having living hinges 156A, 156B and 156C. Living hinges 156A - 156C can divide sidewall 154 into panels 158A and 158B. Body 152 can further comprise proximal end portion 160 and distal end portion 162. Body 152 can have an outer diameter D5 that forms the largest outer perimeter of deployable bone anchor 150 in the non-expanded state of FIG. 3. FIG. 4 is a cross-sectional view of deployable bone anchor 150 of FIG. 3 showing humeral head attachment member 164 and deployment device attachment member 166. Humeral head attachment member 164 can comprise end face 168, cylinder 172 and socket 173, and deployment device attachment member 166 can comprise end face 170, cylinder 174 and bore 175. FIGS. 3 and 4 are discussed concurrently.

Distal end portion 160 can be considered distal for being the portion of bone anchor 150 configured to be inserted into bone. Proximal end portion 162 can be considered proximal for being the portion of bone anchor 150 configured to be exposed for attachment to a prosthetic device.

Body 152 can be fabricated of a biocompatible material, such as metal or polymer. In examples, body 152 can comprise a hybrid construction with metal components and polymer components. For example, flexible components of body 152 can be made of polymer while rigid components of body 152 can be made of metal. In examples, panels 158A and 158B, proximal end portion 160, distal end portion 162, attachment member 164 and attachment member 166 can be fabricated from metal and living hinges 156A, 156B and 156C can be made of polymer. In examples, panels 158A and 158B, proximal end portion 160, distal end portion 162, attachment member 164 and attachment member 166 can be fabricated from polymer and living hinges 156A, 156B and 156C can be made of thinner sections of polymer to facilitate flexure. For example, thickness T1 and T2 can be smaller than thickness T3. In additional examples, thickness T1 of living hinges 156A and 156B can be different than the thickness T2 for living hinge 156C to facilitate bending of living hinges 156A and 156B before living hinge 156C.

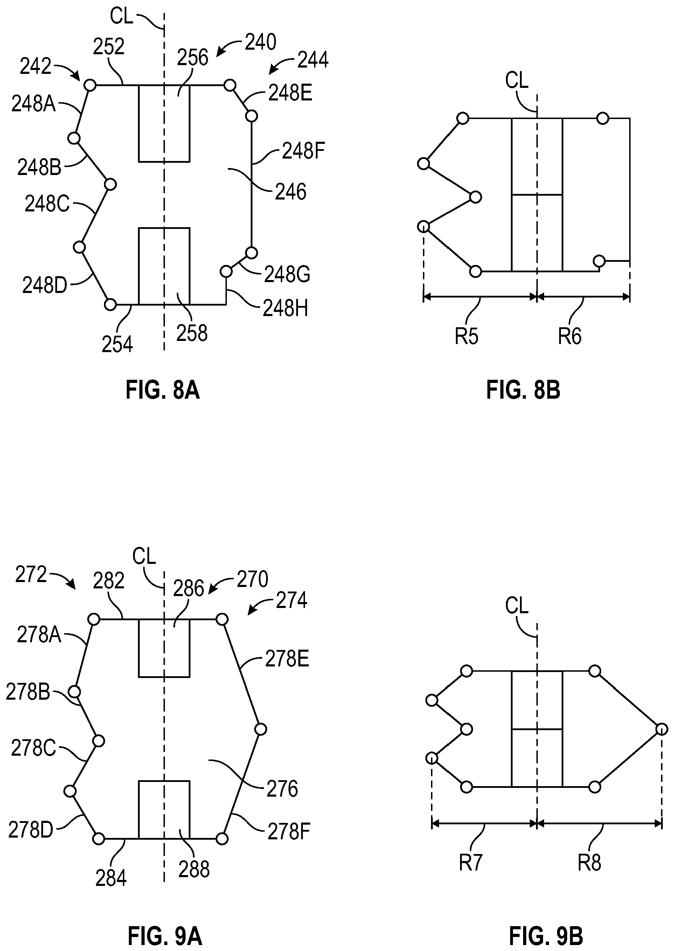

Body 152 can be uniform about center line CL. That is, the cross-section of body 152 visible in FIG. 4 can be revolved about center line CL such that living hinges 156A -156C and panels 158A and 158B comprise hoop-like structures. However, as discussed with reference to FIGS. 8A - 10B, body 152 can be non-uniform with reference to center line CL to, for example, produce radially variable bodies.

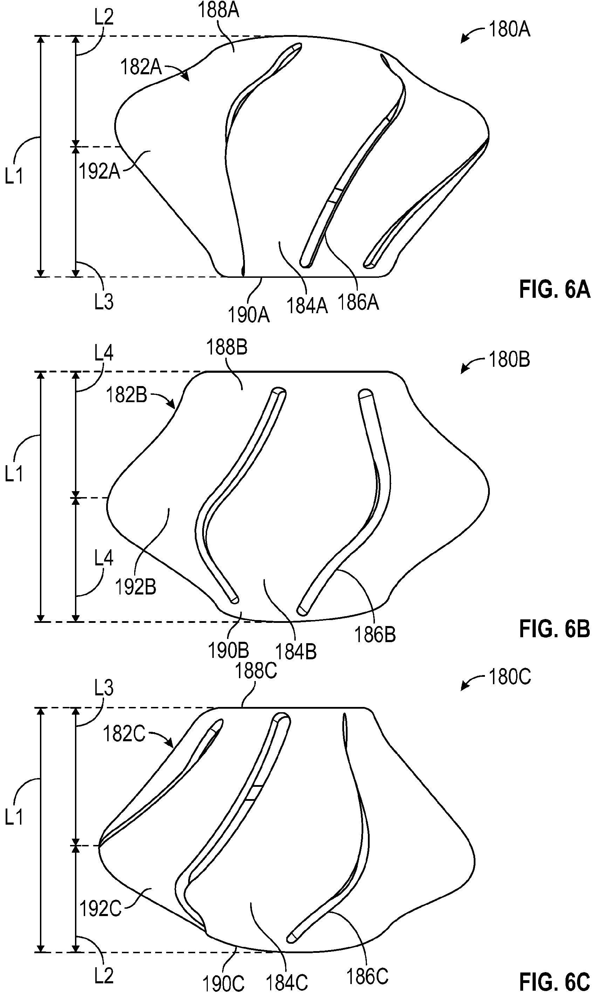

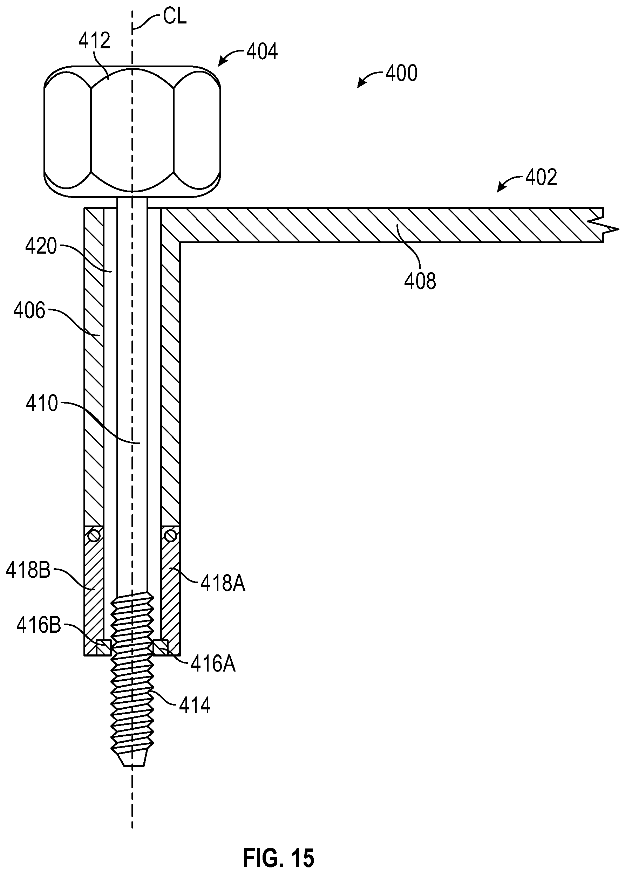

Diameter D5 (FIG. 3) can be configured to be approximately the same as diameter D3 (FIG. 2C) so that deployable bone anchor 150 can fit into bore 110 (FIG. 2B). Diameter D5 can be selected to that deployable bone anchor 150 can be press-fit into bore 110 or bone bore 110 can be produced to form a press-fit with body 152. Deployable bone anchor 150 can thus be positioned so that living hinge 156C is below plane 108 (FIG. 2B). As discussed with reference to deployment device 400 of FIG. 15, a deployment device can be attached to deployable bone anchor 150 to flex living hinges 156A - 156C and bring end face 168 into engagement with end face 170, such that distance D6 (FIG. 4) is zero, which can cause radii R1 and R2 to increase and radius R3 to decrease. Thus, panels 158A and 158B can be oriented radially outward to cause a bowing or enlargement of sidewall 154, such as shown in FIG. 6B.

Humeral head attachment member 164 can comprise socket 173 within cylinder 172 into which a projection or stem from a prosthetic component can extend. In examples, socket 173 can comprise a bore having a frustoconical shape to form a Morse taper. However, in various examples, socket 173 can utilize any suitable feature for coupling to another component, such as a threaded bore. In examples, cylinder 172 can provide a passage through proximal end portion 160 to reach distal end portion 162.

Deployment device attachment member 166 can comprise bore 175 within cylinder 174 into which a deployment device, such as deployment device 400 of FIG. 15, can be inserted. In examples, bore 175 can be threaded. However, in various examples, deployment device attachment member 166 can utilize any suitable feature for coupling to another component. In examples, cylinder 174 can provide a passage through distal end portion 162.

Cylinder 172, or another portion of body 152, can form end face 168. End face 168 can be located a fixed distance from proximal end portion 160. Cylinder 174, or another portion of body 152, can form end face 170. End face 170 can be located a fixed distance D6 from distal end portion 162 in the undeployed state. The distances between end face 168 and proximal end portion 160 and end face 170 and distal end portion 162 can be equal or different. The distances between end face 168 and proximal end portion 160 and end face 170 and distal end portion 162 can be selected to allow for proximal end portion 160 and distal end portion 162 to be brought closer a distance equal to distance D6. Distance D6 can be selected to allow living hinges 156A - 156C to flex a desired amount to control the amount of radial expansion of deployable bone anchor 150. Shortening of distance D6, such as via a deployment device, can thus change diameter D5 (FIG. 3) or diameter D4 of perimeter 130 (FIG. 2C). The smaller distance D6 is, the smaller diameter D4 will be. The larger distance D6 is, the larger diameter D4 will be. The largest diameter D4 can correspond to a distance D6 equaling zero when panels 158A and 158B contact each other.

In the example of FIG. 4, the lengths of panels 158A and 158B can be equal such that living hinge 156C is positioned centrally between end portion 160 and end portion 162. As such, the location of perimeter 130 (FIG. 2C) can be axially equidistant between end portion 160 and end portion 162, similar to what is shown in FIG. 6B.

In examples, the length of panel 158A can be longer than the length of panel 158B such that living hinge 156C is positioned closer to end portion 162. As such, the location of perimeter 130 (FIG. 2C) can be axially closer to end portion 162, similar to what is shown in FIG. 6C.

In examples, the length of panel 158B can be longer than the length of panel 158A such that living hinge 156C is positioned closer to end portion 160. As such, the location of perimeter 130 (FIG. 2C) can be axially closer to end portion 160, similar to what is shown in FIG. 6A.

FIG. 5 is a side view of deployable bone anchor 150 of FIG. 3 with the addition of porous structures 176A and 176B on sidewall 154. Deployable bone anchor 150 of FIG. 5 can be configured similarly as deployable bone anchor 150 of FIG. 3, with porous structures 176A and 176B added to panels 158A and 158B, respectively. Porous structures 176A and 176B can comprise coatings applied to panels 158A and 158B, porous bodies embedded into pockets within panels 158A and 158B, or surfaces of panels 158A and 158B when panels 158A and 158B are fabricated from porous material. In examples, porous structures 176A and 176B can comprise a Cancellous-Structured Titanium^{™} (CSTi^{™}) layer, for example. CSTi^{™} porous layers are manufactured by Zimmer, Inc., of Warsaw, Ind. Cancellous-Structured Titanium^{™} and CSTi^{™} are trademarks of Zimmer, Inc. In examples, porous structures 176A and 176B can comprise Trabecular Metal^{™} technology generally available from Zimmer, Inc., of Warsaw, Ind. Trabecular Metal^{™} is a trademark of Zimmer, Inc. Such a material can be formed from a reticulated vitreous carbon foam substrate which is infiltrated and coated with a biocompatible metal, such as tantalum, by a chemical vapor deposition ("CVD") process in the manner disclosed in detail in U.S. Pat. No. 5,282,861. In addition to tantalum, other metals such as niobium, or alloys of tantalum and niobium with one another or with other metals may also be used. In additional examples of the present disclosure, porous bodies and surfaces can be located on other portions of deployable bone anchor 150.

Although not illustrated in FIGS. 3 and 5, body 152 can comprise slits similar to slits 116A - 116C of FIG. 2A and 2B or other openings to facilitate flexure of sidewall 154. Such slits can be axially aligned with center line CL between end portion 160 and end portion 162. In examples, such slits can be transverse to center line CL between end portion 160 and end portion 162. In examples, such slits can extend all the way between end portion 160 and end portion 162 to have the same or similar lengths as sidewall 154. In examples, such slits can be less than the distance between end portion 160 and end portion 162.

FIGS. 6A, 6B and 6C are side views of deployable bone anchors 180A, 180B and 180C of the present disclosure in top-heavy, symmetric, and bottom-heavy deployment configurations. Deployable bone anchors 180A, 180B and 180C can be configured similarly as deployable bone anchor 100 of FIGS. 2A - 2C or deployable bone anchor 150 of FIGS. 3 - 5. FIGS. 6A - 6C illustrate that the deflection point for radially expansion of the device can be located in different axial positions for different examples or embodiments of the device. Deployable bone anchors 180A, 180B and 180C can be deployed such that the axial length of each device collapses to a shorter length, such as where attachment member 164 contacts attachment member 166 within sidewall 154 for the examples of FIGS 3 - 5, thereby producing a bulge in the sidewall. As such, axial compression of bone anchors 180A - 180C can cause radial expansion of bone anchors 180A - 180C and axial expansion of bone anchors 180A - 180C can cause radial retraction of bone anchors 180A - 180C. FIGS. 6A - 6C illustrate that the axial location of the radial bulge relative to the axial length of the device can be varied.

FIG. 6A illustrates top-heavy deployable bone anchor 180A comprising body 182A having flexible panels 184A that can be formed by slits 186A. Body 182A can extend between proximal end 188A and distal end 190A. Flexible panels 184A can be configured be bend at a location between proximal end 188A and distal end 190A to form rim 192A. In the example of FIG. 6A, rim 192A can be located in the proximal-most half of body 182A. As such, the distance between proximal end 188A and rim 192A can be L2 and the distance between distal end 190A and rim 192A can be L3, wherein L3 is greater than L2.

FIG. 6B illustrates a symmetric deployable bone anchor 180B comprising body 182B having flexible panels 184B that can be formed by slits 186B. Body 182B can extend between proximal end 188B and distal end 190B. Flexible panels 184B can be configured be bend at a location between proximal end 188B and distal end 190B to form rim 192B. In the example of FIG. 6B, rim 192B can be located in the center portion of body 182B. As such, the distance between proximal end 188B and rim 192B can be L4 and the distance between distal end 190B and rim 192B can be L4.

FIG. 6C illustrates bottom-heavy deployable bone anchor 180C comprising body 182C having flexible panels 184C that can be formed by slits 186C. Body 182C can extend between proximal end 188C and distal end 190C. Flexible panels 184C can be configured be bend at a location between proximal end 188C and distal end 190C to form rim 192C. In the example of FIG. 6C, rim 192C can be located in the distal-most half of body 182C. As such, the distance between proximal end 188C and rim 192C can be L3 and the distance between distal end 190C and rim 192C can be L2, wherein L3 is greater than L2.

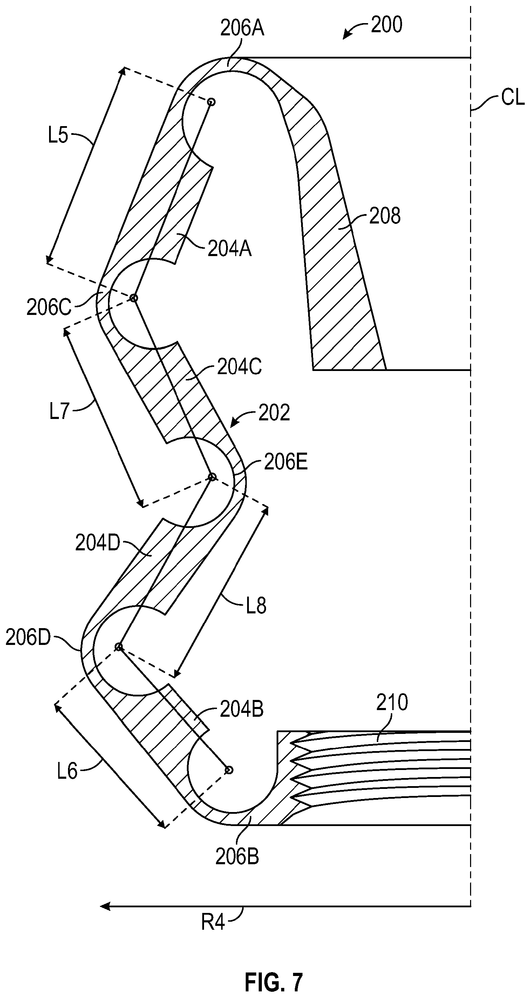

FIG. 7 is a side cross-sectional view of a portion of deployable bone anchor 200 comprising flexible sidewall 202 of the present disclosure comprising moveable panels 204A, 204B, 204C and 204D and living hinges 206A, 206B, 206C, 206D and 206E. Moveable panels 204A, 204B, 204C and 204D can have lengths L5, L6, L7 and L8, respectively. Flexible sidewall 202 can be configured to collapse into a W shape when attachment member 208 contacts attachment member 210. Flexible sidewall 202 can have a symmetric shape about center line CL.

Deployable bone anchor 200 can be configured similarly to deployable bone anchor 150 of FIG. 4 with living hinge 156C being replaced by panels 204C and 204D and living hinges 206C - 206E.

Living hinges 206A, 206C, 206D and 206B can face radially inward and living hinge 206E can face radially outward. Living hinges 206C - 206E can be configured become smaller as deployable bone anchor 200 is deployed or made axially shorter, while living hinges 206A and 206B can be configured to become larger as deployable bone anchor 200 is deployed or made smaller. As such, in the deployed state, deployable bone anchor 200 can have a W shape when attachment member 208 is brought into engagement with attachment member 210.

In the undeployed or undeflected state, deployable bone anchor 200 can have radius R4 relative to center line CL. Radius R4 can be located at both of living hinges 206C and 206D.

As attachment member 208 is brought closer to attachment member 210 during deployment of deployable bone anchor 200, living hinges 206A and 206B can rotate outward to bring panels 204A and 204B closer to perpendicular to center line CL, and living hinges 206B - 206E can rotate inward to bring panels 204C and 204D closer to perpendicular to center line CL, thereby increasing radius R4. Simultaneously with movement of panels 204A and 204B, living hinges 206C and 206D can become reduced in size and panels 204C and 204D can be brought closer to panels 204A and 204B, respectively.

In examples, lengths L5 and L6 can be equal to each other and lengths L7 and L8 can be equal to each other. In examples, lengths L5 - L8 can all be equal to each other. However, as discussed herein, Lengths L5 - L8 can be asymmetric so that the radial peaks produced at living hinges 206C and 206D can be different from each other, e.g., the magnitude of radius R4 at living hinges 206C and 206D can be different in the deployed state.

Deployable bone anchors of the present disclosure can be configured to have a variety of different panels, including varying numbers of panels and lengths of panels, as shown in FIGS. 8A - 9B.

FIGS. 8A and 8B are diagrammatic views of asymmetric deployable bone anchor 240 having first sidewall panel 242 that radially expands and second sidewall panel 244 that remains flat upon deployment. Deployable bone anchor 240 can comprise body 246 having flexible panels 248A - 248H, which can be formed by slits similar to slits 116A - 116C of FIGS. 2A and 2B. Body 246 can extend between proximal end 252 and distal end 254. Body 246 can include attachment member 256 and attachment member 258.

First sidewall panel 242 can extend around a first segment of a perimeter or circumference of deployable bone anchor 240 relative to center line CL. Second sidewall panel 244 can extend around a second segment of a perimeter or circumference of deployable bone anchor 240 relative to center line CL. In examples, first sidewall panel 242 and second sidewall panel 244 can each extend around approximately one-hundred-eighty degrees of the perimeter of deployable bone anchor 240.

As can be seen in FIG. 8B, first sidewall panel 242 can deform to form a W shape similar to the configuration of FIG. 7 and second sidewall panel 244 can deform to form a straight line. As such, a segment or one half of deployable bone anchor 240 can form a double pronged body and a segment or one half of deployable bone anchor 240 can form a cylindrical shape. The different sections of body 246 can be separated by slits discussed herein. The prongs formed by panels 248A - 248D can be radially longer than the surface formed by panels 248E - 248H, such that R5 is greater than R6. Thus, the prongs formed by panels 248A -248D can be positioned in anatomy to displace unhealthy bone and the surface formed by panels 248E - 248H can be positioned in anatomy to face healthy bone or a cortical bone feature such as intertubercular groove 132 (FIG. 2C) or a sulcus. However, deployable bone anchor 270 can be positioned in anatomy in any suitable orientation as determined by a surgeon.

FIGS. 9A and 9B are diagrammatic views of asymmetric deployable bone anchor 270 having first sidewall panel 272 that radially expands more than second sidewall panel 274. Deployable bone anchor 270 can comprise body 276 having flexible panels 278A - 278F, which can be formed by slits similar to slits 116A - 116C of FIGS. 2A and 2B. Body 276 can extend between proximal end 282 and distal end 284. Body 276 can include attachment member 286 and attachment member 288.

First sidewall panel 272 can extend around a first segment of a perimeter or circumference of deployable bone anchor 270 relative to center line CL. Second sidewall panel 274 can extend around a second segment of a perimeter or circumference of deployable bone anchor 270 relative to center line CL. In examples, first sidewall panel 272 and second sidewall panel 274 can each extend around approximately one-hundred-eighty degrees of the perimeter of deployable bone anchor 240.

As can be seen in FIG. 9B, first sidewall panel 272 can deform to form a W shape similar to the configuration of FIG. 7 and second sidewall panel 274 can deform to form a V shape. As such, a segment or one half of deployable bone anchor 270 can form a double pronged body and a segment or one half of deployable bone anchor 270 can form a single pronged body. The different sections of body 276 can be separated by slits discussed herein. The prongs formed by panels 278A - 278D can be radially shorter than the prong formed by panels 278E and 278F, such that radius R8 can be greater than radius R7. Thus, the prongs formed by panels 278A - 278D can be positioned in anatomy to face a cortical bone wall and the prong formed by panels 278E and 278F can be positioned in anatomy to face healthy bone. However, deployable bone anchor 270 can be positioned in anatomy in any suitable orientation as determined by a surgeon.

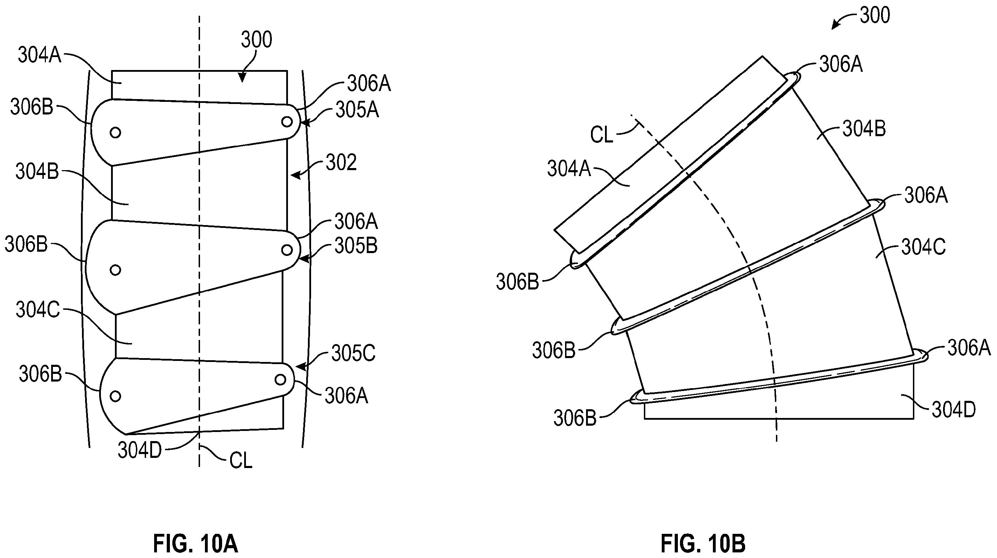

FIG. 10A is a diagrammatic side view of deployable bone anchor 300 of the present disclosure comprising sidewall 302 having variable width panels 304A - 304D that can induce curvature of bone anchor 300 in a deployed state. Sidewall 302 can have living hinges 305A, 305B and 305C, which can include first living hinge section 306A and second living hinge section 306B. FIG. 10B is a diagrammatic side view of deployable bone anchor 300 of FIG. 10A in the deployed state such that center line CL of bone anchor 300 is curved.

Variable width panels 304A - 304D can have various geometric shapes, including rectangular, trapezoidal and triangular. The axial heights of variable width panels 304A - 304D can be different on opposite circumferential sides of sidewall 302. For example, the left side of panels 304B and 304C in FIG. 10A can be shorter than the right side of panels 304B and 304C.

First living hinge section 306A and second living hinge section 306B can form opposite ends of living hinges 305A -305C and can have various geometric shapes, including rectangular, trapezoidal and triangular. The living hinges formed by first living hinge section 306A and second living hinge section 306B can have axial heights that are different on opposite circumferential sides of sidewall 302. For example, the first living hinge sections 306B in FIG. 10A can be taller than second living hinge sections 306A. In other words, first living hinge sections 306A can have smaller radii of curvature than second living hinge sections 306B.

The axial heights of variable width panels 304A - 304D and the living hinges formed by first living hinge section 306A and second living hinge section 306B can be arranged in opposite circumferential positions such that, in a non-deployed state, deployable bone anchor 300 can have a generally cylindrical shape to fit within a bone bore or canal, such as bore 110 (FIG. 2A). However, because the differing axial heights of the living hinges formed by first living hinge section 306A and second living hinge section 306B, compression of sidewall 302 via the deployment actions described herein can cause one circumferential side of deployable bone anchor 300 to contract a greater axial distance than the opposite circumferential side. Thus, with reference to FIG. 10B, the left side of deployable bone anchor 300 will contract a greater amount than the right side because the axial lengths of second living hinge sections 306B are greater than the axial lengths of first living hinge sections 306A.

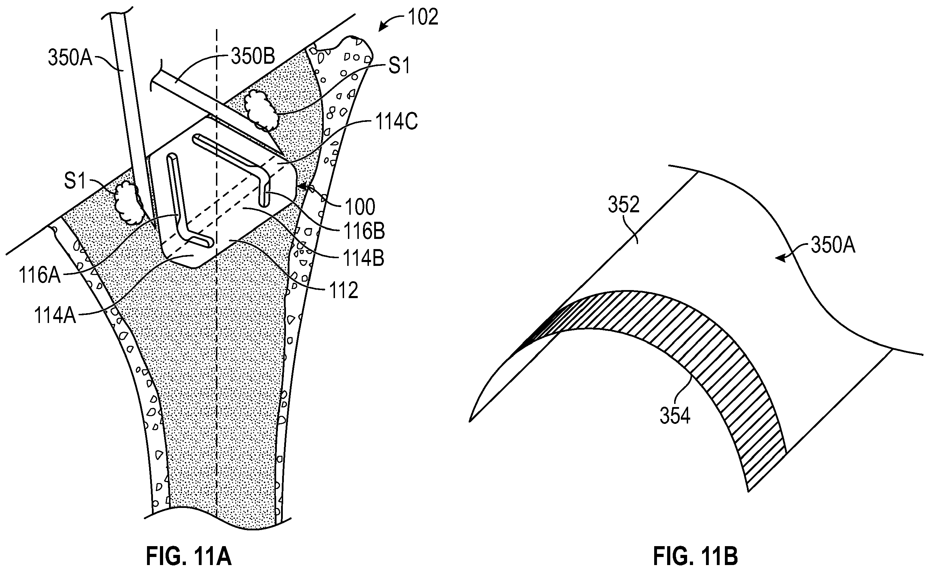

FIG. 11A is a diagrammatic side view of deployable bone anchor 100 of the present disclosure being separated from bone matter within humerus 102 with tools 350A and 350B. FIG. 11B is a perspective view of a distal end of tool 350A of FIG. 11A comprising shaft 352 and tip 354.

The prosthetic component attached to bone anchor 100 can become worn or ineffective, or the shoulder joint into which bone anchor 100 has been implanted can change or further degrade to a point where it is desirable to perform a revision procedure where a different prosthetic device is implanted in the shoulder joint. As such, bone anchor 100 can be removed from humerus 102 to allow for the placement of a new device. After being implanted within humerus 102 for an extended period of time, cancellous bone matter can grow into porous structures on bone anchor 100 or into slits 116A and 116B. Typically, such boney ingrowth is advantageous to hold bone anchor 100 in place. However, when a revision procedure is performed it becomes desirable to separate the cancellous bone from the bone anchor 100 to prevent or limit damaging the healthy cancellous bone surrounding bone anchor 100.

In order to remove, bone anchor 100 from humerus 102 and to preserve bone, bone anchor 100 can be axially expanded in order to radially shrink the footprint of bone anchor 100 to allow bone anchor 100 to be axially pulled from humerus 102 without displacing sections S1 of healthy bone. As discussed with reference to FIG. 15, deployment device 400 can be attached to bone anchor 100 to cause axial contraction/radial expansion for implantation and axial expansion/radial contraction for removal. Before deployment device 400 is used, it can be advantageous to first break the connection of bone anchor 100 to cancellous bone formed by the boney ingrowth to allow bone anchor 100 to be radially collapsed without disrupting sections S1.

In the example of FIGS. 11A and 11B, tools 350A and 350B can comprise osteotome gouges. Tool 350A can comprise shaft 352 which can have an axial length sufficient to allow tool 350A to be extended into anatomy of a surgical sight. Shaft 352 can be curved to fit around the curvature of bone anchor 100. Tip 354 of shaft 352 can be sharpened or serrated to cut through cancellous bone. Tool 350B can be configured similarly as tool 350A.

Tools 350A and 350B can be slid along surfaces of bone anchor 100 to slice or otherwise separate cancellous bone away from bone anchor 100. As such, body 112 can be more readily collapsed in the radial direction with deployment device 400 without having to overcome the force needed to break bone matter.

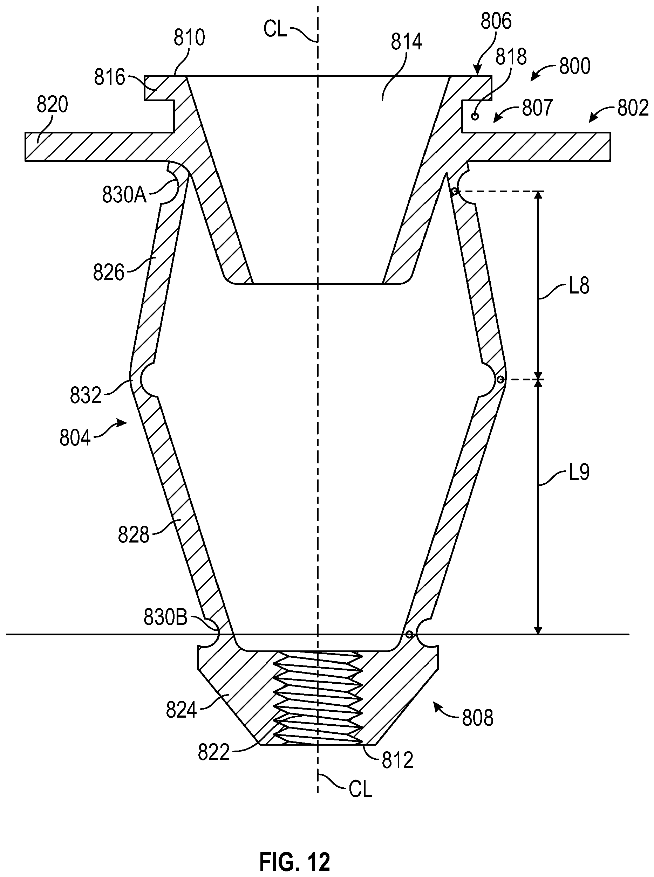

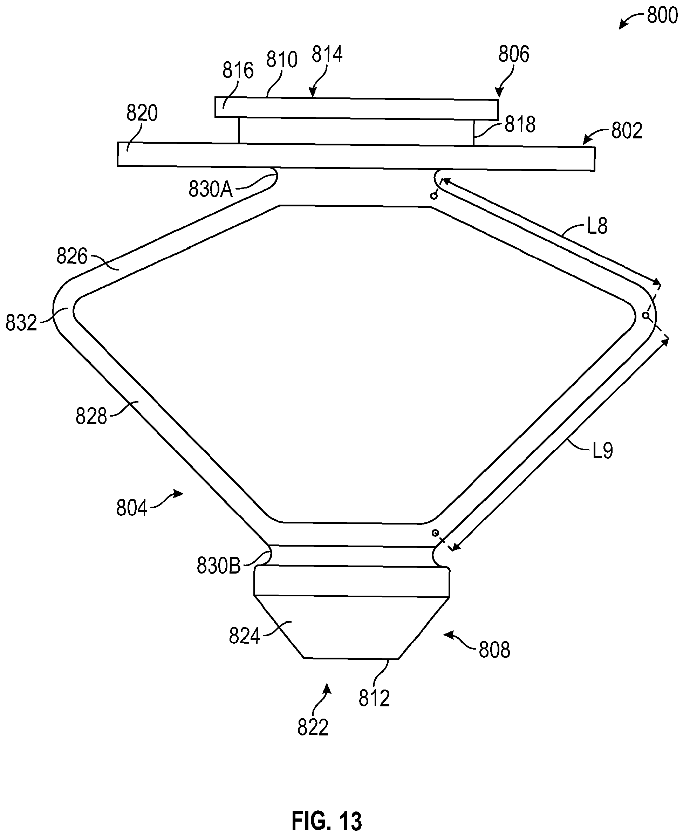

FIG. 12 is a side cross-sectional view of deployable bone anchor 800 of the present disclosure having features for engaging deployment device 400 of FIG. 15. FIG. 13 is a side cross-sectional view of deployable bone anchor 800 of FIG. 12 in an expanded state. FIGS. 12 and 13 are discussed concurrently. The features for engaging deployment device 400 can be incorporated into any of the deployable bone anchors and trauma rods described herein.

Bone anchor 800 comprises body 802, sidewall 804, first attachment member 806, second attachment members 807, third attachment member 808, proximal end 810 and distal end 812.

First attachment member 806 can comprise a component for attaching to a prosthetic component such as a humeral head. Attachment member 806 can comprise socket 814. Socket 814 can comprise a Morse taper socket comprising a frustoconical sleeve configured to receive a mating component attached to a prosthetic component.

Second attachment member 807 can comprise a component for attaching to a deployment device, such as deployment device 400 of FIG. 15. Attachment member 807 can comprise flange 816, undercut 818 and disk 820.

Third attachment member 808 can comprise a component for connecting to a deployment device, such as deployment device 400 of FIG. 15. Attachment member 806 can comprise bore 822 and tip 824.

Sidewall 804 can comprise proximal panel 826 and distal panel 828, living hinges 830A and 830B and living hinge 832. Proximal panel 826 can have length L8 and distal panel 828 can have length L9. In the illustrated example, length L9 is greater than length L8. However, length L8 can be greater than length L9 and lengths L8 and L9 can be equal in other configurations. Sidewall 804 can function as any of the deflectable panels and living hinges described herein to, for example, radially expand when subject to compressive forces applied by, for example, deployment device 400 of FIG. 15.

Second attachment member 807 and third attachment member 808 can cooperate to attach to different portions of a deployment device. Flange 816 can form undercut 818 that can allow a sleeve of a deployment device to attach to bone anchor 800. Bore 822 can comprise a threaded bore to attach to a drive shaft of a deployment device. The deployment device can be configured to bring tip 824 and socket 814 closer together. In examples, both attachment member 807 and attachment member 808 can provide axial interlocking with the deployment device to allow axial force to be transmitted between tip 824 and socket 814. FIGS. 12 and 13 show one particular configuration for attachment members 807 and 808, though others can be used.

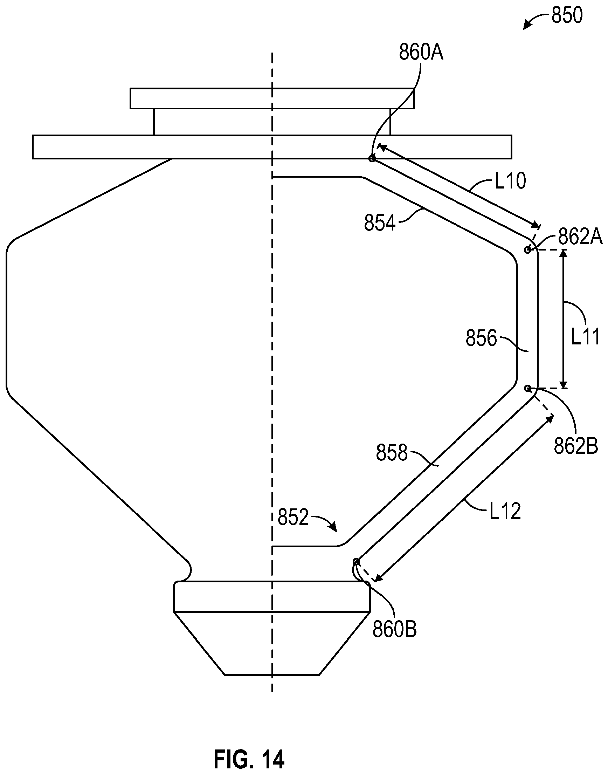

FIG. 14 is a side cross-sectional view of deployable bone anchor 850 configured to engage a deployment device. Bone anchor 850 can be configured similarly as bone anchor 800 of FIGS. 12 and 13. However, sidewall 804 including proximal panel 826, distal panel 828, living hinges 830A and 830B and living hinge 832 can be replaced with sidewall 852 including proximal panel 854, middle panel 856, distal panel 858, living hinges 860A and 860B and living hinges 862A and 862B. Proximal panel 854 can have length L10, middle panel 856 can have length L11 and distal panel 858 can have length L12. In the illustrated example, length L10 is greater than length L11 and length L12 is greater than length L10, through other configurations can be used. Sidewall 852 can be configured to produce a truncated V shape when deployed. In examples, living hinges 860A and 860B can be formed of portions of sidewall 852 that are thinner than portions of sidewall 852 forming living hinges 862A and 862B, thereby causing living hinges 860A and 860B to deflect before living hinges 862A and 862B, which can ensure that panels move radially outward upon first deflection.

FIG. 15 is a partial cross-sectional view of deployment device 400 of the present disclosure comprising sleeve 402 and drive shaft 404. Sleeve 402 can comprise tube 406 and handle 408. Drive shaft 404 can comprise shank 410, knob 412 and threaded tip 414. Tube 406 can comprise teeth 416A and 416B. Deployment device 400 can be used to deploy and retrieve deployable bone anchors of the present disclosure.

Teeth 416A and 416B can be configured to engage attachment member 807. In particular, teeth 416A and 416B can be positioned in undercut 818 underneath flange 816. Teeth 416A and 416B can be attached to arms 418A and 418B, respectively, in tube 406 that can splay radially outward from the central axis of deployment device 400 to allow tube 406 to fit over flange 816. In examples, arms 418A and 418B can be flexible portions of tube 406 or can be pin-mounted to tube 406 and biased radially inward via springs. Once positioned within undercut 818, teeth 416A and 416B can be configured to prevent deployment device 400 from axially separating from bone anchor 800. Teeth 416A and 416B can rotate about flange 816 to allow handle 408 to be positioned in different circumferential positions relative to bone anchor 800.

Shank 410 of drive shaft 404 can be inserted into passage 420 of tube 406. Threaded tip 414 can be extended beyond teeth 416A and 416B to allow threaded tip 414 to engage threaded bore 822. Knob 412 can be rotated to threadedly engage threaded tip 414 with threaded bore 822. As such, drive shaft 404 and bone anchor 800 can be axially engaged.

With deployment device 400 and bone anchor 800 engaged, a user can apply downward axial pressure to knob 412, thereby keeping bone anchor 800 positioned in bore 110 (FIG. 2A), for example. Knob 412 can be rotated while handle 408 is held to prevent rotation of tube 406 about the central axis of drive shaft 404. Rotation of shank 410 can cause threaded tip 414 to advance further into and through threaded bore 822. Shank 410 can pull knob 412 into engagement with tube 406. Knob 412 can thus push tube 406 downward against disk 820. In other words, the distance between teeth 416A and 416B and knob 412 can decrease such that the distance between tip 824 and disk 820 also must decrease, thereby causing flexure of living hinges 830A, 830B and 832. Knob 412 can be rotated in the opposite direction to pull flange 816 away from tip 824 to axially expand bone anchor 800 in an opposite manner.

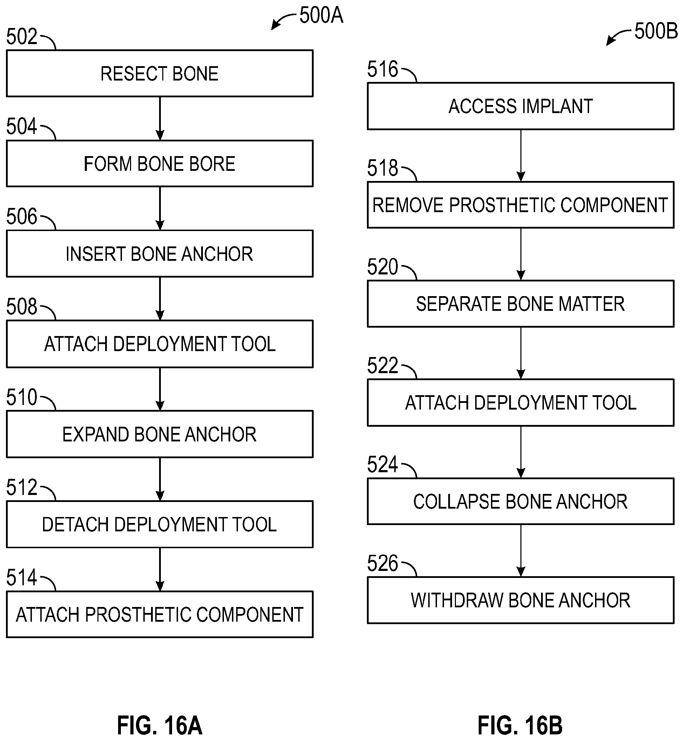

FIG. 16A is a block diagram illustrating steps of method 500A for implanting deployable bone anchors of the present disclosure using deployment devices. FIG. 16B is block diagram illustrating steps of method 500B for removing deployable bone anchors of the present disclosure using deployment devices.

At step 502, a bone can be prepared to receive a prosthetic implant. For example, humerus 102 (FIG. 2A) can be resected at humeral head 106.

At step 504, a bore or canal can be formed in the bone to receive a bone anchor of the prosthetic implant. For example, bore 110 (FIG. 2A) can be formed into plane 108 of the resected humeral head.

At step 506, a deployable bone anchor of the prosthetic implant can be inserted into the bore or canal formed at step 504. For example, bone anchor 800 (FIG. 12) can be inserted into bore 110.

At step 508, a deployment device can be attached to the bone anchor inserted into the bone at step 506. For example, deployment device 400 (FIG. 15) can be attached to bone anchor 800. In particular, teeth 416A and 416B can be attached to flange 316, and threaded tip 414 can be engaged with threaded bore 822.

At step 510, the bone anchor of steps 506 and 508 can be deployed. In particular, the bone anchor can be radially expanded to push portions of the bone anchor into engagement with bone matter, such as cancellous bone. For example, knob 412 can be rotated to cause living hinges 830A, 830B, and 832 to flex and panels 826 and 828 to become more oblique to center line CL. Bone anchor 800 can become axially compressed until a stop feature engages to provide an indication to the user that the bone anchor is fully deployed. For example, the distal end of socket 814 can axially engage the proximal end of tip 824, thereby preventing any further axial compression of bone anchor 800.

At step 512, the deployment device can be detached from the bone anchor. For example, deployment device 400 can be detached from bone anchor 800 by pulling teeth 416A and 416B away from flange 816. In examples, deployment device 400 can include a button to splay arms holding teeth 416A and 416B to facilitate detachment.

At step 514, a prosthetic component can be attached to the bone anchor secured to the bone in steps 504 - 510. For example, humeral head 121 (FIG. 2B) can be attached to bone anchor 800 by insertion of coupler 122 into socket 814.

As such, bone anchor 800 and humeral head 121 can remain implanted in anatomy of a patient to provide an artificial shoulder joint. Over time, the shoulder joint can become further degraded such that humeral head 121 is not effective or humeral head 121 can be ill-suited for the performance expectations of the patient. As such, it can be desirable to replace humeral head 121 and bone anchor 800.

At step 516, a previously implanted bone anchor can be accessed within anatomy. For example, soft tissue can be opened up to expose humeral head 121.

At step 518, a prosthetic component can be removed from the implanted bone anchor. For example, humeral head 121 can be removed from bone anchor 800. For example, coupler 122 can be withdrawn from socket 814.

At step 520, bone matter attached to the previously implanted bone anchor can be separated from the bone anchor. In particular, cancellous bone matter grown into the bone anchor can be broken. In examples, osteotome gouges, such as tools 350A and 350B, can be slid along bone anchor 800 to separate bone matter therefrom.

At step 522, a deployment device can be attached to the deployed bone anchor. For example, deployment device 400 can be attached to bone anchor 800 in a manner similar to step 508.

At step 524, the previously implanted bone anchor can be radially collapsed such as via axial expansion. In particular, the previously implanted bone anchor can be radially collapsed to withdraw portions of the bone anchor from bone matter. For example, knob 412 can be rotated to cause living hinges 830A, 830B, and 832 to flex and panels 826 and 828 to become more parallel to center line CL. Bone anchor can be radially collapsed until sufficient clearance from or less interference with bone matter is obtained or until bone anchor 800 is fully collapsed.

At step 526, the bone anchor can be removed from the bone. For example, bone anchor 800 can be axially pulled out of humerus 102 (FIG. 2A).

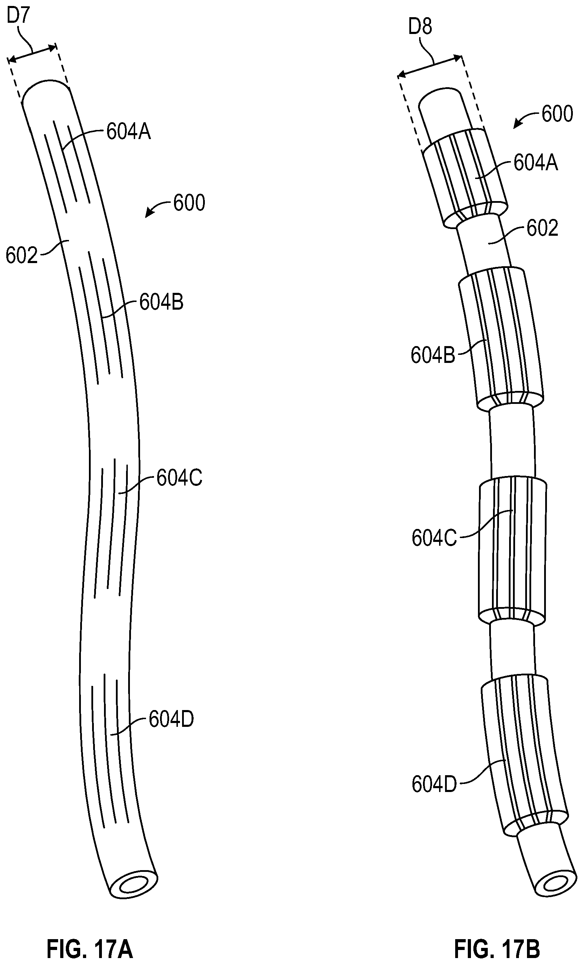

FIG. 17A is a schematic view of deployable trauma rod 600 of the present disclosure including shaft 602 with a plurality of radially expandable zones 604A, 604B, 604C and 604D. FIG. 17B is a schematic view of the deployable trauma rod of FIG. 17A in a deployed state such that the radially expandable zones 604A - 604D are radially enlarged. FIGS. 17A and 17B are discussed concurrently.

As can be seen in FIG. 17A, shaft 602 can have diameter D7 and expandable zones 604A, 604B, 604C and 604D can be collapsed to have diameters equal to diameter D7. However, in FIG. 17B, expandable zones 604A, 604B, 604C and 604D can be radially expanded to diameter D8, which is greater than diameter D7. In examples, deployable trauma rod 600 can be fabricated of NiTinol.

Expandable zones 604A, 604B, 604C and 604D can be configured to have living hinges, deflectable panels and slits as described herein. Deployable trauma rod 600 can be attached to deployment devices described herein to expand and contract radially expandable zones 604A - 604D.

In examples of deployable trauma rod 600, a tensioning shaft can be left inside shaft 602. For example, drive shaft 404 (FIG. 15) can be configured so that a lower portion of shank 410 can remain attached to shaft 602 with the upper portion of shank 410 being removable. The lower portion of shank 410 can additionally be attached to a proximal end of deployable trauma rod 600 (with threaded tip 414 being attached to the distal end) to keep compression applied to deployable trauma rod 600.

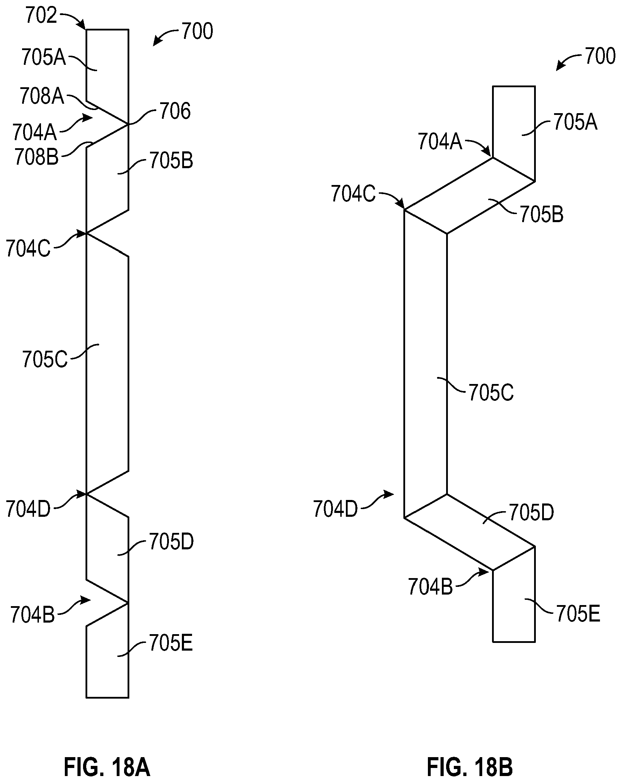

FIG. 18A is a schematic view of deployable bone anchor 700 comprising sidewall 702 including living hinges 704A, 704B, 704C and 704D having hard stops integrated therein. FIG. 18B is a schematic view of deployable bone anchor 700 of FIG. 18A in a deployed state such that the integrated hard stops are engaged. FIGS. 18A and 18B are discussed concurrently. Sidewall 702 can be used in any of the bone anchors or trauma rods described herein. Living hinges 704A, 704B, 704C and 704D can connect panels 705A, 705B, 705C, 705D, 705E and 705F. Living hinge 704A can include flexible portion 706 and surfaces 708A and 708B that form an integrated stop. Surfaces 708A and 708B and flexible portion 706 can be monolithic with the material of sidewall 702. Living hinges 704B - 704D can include similar features, but description of such is omitted for brevity. Living hinges 704A and 704B can face outward and living hinges 704C and 704D can face inward. When deployable bone anchor 700 is subject to axially compressive forces living hinges can flex to move panel 705C radially outward to the configuration of FIGS. 18B where sidewall 702 is radially expanded. Axially compression of sidewall 702 can be arrested or impeded by engagement of surfaces 708A and 708B. Surfaces 708A and 708B can be connected by flexible portion 706 and angled relative to each other in the relaxed or non-deployed state to limit axial movement of panel 705A toward panel 705E in the compressed or deployed state. The angle between surfaces 708A and 708B can be set at various angles based on design needs.

In examples of the present disclosure, binder jetting manufacturing processes can be used to fabricated any of the deployable bone anchors or trauma rods described herein. Binder jetting processes can involve the use of a binding adhesive agent onto layers of a powdered material. The process can use a head similar to an inkjet printing head to dispense a layer of adhesive onto layers of powder of the material spread onto a platform.

Optionally the living hinge portion comprises: a first portion of the sidewall proximate the first end; and a first flex feature connecting the first portion to the first end; wherein the first portion of the sidewall includes an axially variable height; and wherein the first flex feature includes an axially variable height.

Described herein is a system for attaching an orthopedic implant to bone, the system comprising: a deployment device comprising: a sleeve extending along an axis; and a drive shaft extending through the sleeve; and a deployable bone anchor comprising: a first end comprising: a socket for receiving a mating feature of a prosthetic component; and a coupler for attaching to the sleeve along the axis; a second end for insertion into a bone, the second end comprising a bore for receiving the drive shaft; and a sidewall connecting the first end and the second end, the sidewall comprising: a plurality of axially extending panels; and a plurality of living hinges to allow the plurality of axially extending panels to rotate radially relative to the axis.

Optionally the sleeve comprises a tube having an internal passage.

Described herein is a method of implanting a prosthetic component in a bone, the method comprising: forming a bore in a resected surface of a bone; inserting a distal end of a deployable bone anchor into the bore; attaching a deployment device to a proximal end of the deployable bone anchor; radially expanding the deployable bone anchor with the deployment device; detaching the deployment device; and attaching a prosthetic component to the deployable bone anchor.

Optionally inserting the distal end of the deployable bone anchor into the bore comprises inserting the deployable bone anchor into the bore along an axis.

Optionally inserting the distal end of the deployable bone anchor into the bore comprises protruding a proximal end of the deployable bone anchor outside of the bore.

Optionally attaching the deployment device to the deployable bone anchor comprises: attaching a sleeve to a proximal end of the deployable bone anchor; and attaching a drive shaft extending through the sleeve to the distal end of the deployable bone anchor.

Optionally attaching the sleeve to the proximal end of the deployable bone anchor comprises deflecting a plurality of teeth attached to the sleeve to fit around a flange of the deployable bone anchor; and attaching the drive shaft extending through the sleeve to the distal end of the deployable bone anchor comprises threading a distal end of the drive shaft into a threaded bore of the deployable bone anchor.

In Optionally radially expanding the deployable bone anchor with the deployment device comprises axially compressing the deployable bone anchor with the deployment device.

Optionally radially expanding the deployable bone anchor with the deployment device comprises arresting further compressing of the deployable bone anchor by engaging a first stop extending axially from the proximal end into the deployable bone anchor with a second stop extending axially from the distal end into the deployable bone anchor.

Optionally radially expanding the deployable bone anchor with the deployment device comprises flexing a plurality of living hinges.

In Optionally radially expanding the deployable bone anchor with the deployment device comprises inducing axial curvature in the deployable bone anchor.

Optionally radially expanding the deployable bone anchor with the deployment device comprises radially expanding the deployable bone anchor differently at different axial locations along or different circumferential locations around the deployable bone anchor.

### Various Notes

The above detailed description includes references to the accompanying drawings, which form a part of the detailed description. The drawings show, by way of illustration, specific embodiments in which the invention can be practiced.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A deployable bone anchor for an orthopedic implant, the deployable bone anchor comprising:
an implantable body (154) comprising:
a first end comprising:
a first attachment member (806) to facilitate coupling of a prosthetic component to the implantable body; and
a second attachment member (807) to facilitate coupling of a deployment device to the implantable body;
a second end for insertion into a bone, the second end comprising a third attachment member (808) to facilitate coupling to the deployment device; and
a sidewall (154, 202, 302, 702, 804, 852) connecting the first end and the second end, the sidewall comprising:
a living hinge portion configured to allow the sidewall to flex when the second attachment member (807) and the third attachment member (808) are pulled toward each other by the deployment device when the deployment device is attached to the implantable body;
wherein the sidewall (154, 202, 302, 702, 804, 852) is configured to deploy from an extended configuration where the sidewall is axially extending to a collapsed configuration where the sidewall is bowed radially outward; and
wherein the living hinge portion is configured to induce curvature to the sidewall in the collapsed configuration.

2. The deployable bone anchor of claim 1, wherein:
the sidewall (154, 202, 302, 702, 804, 852) comprises a plurality of slits (116A, 116B, 186A, 186B, 186C) extending through the sidewall; and
the plurality of slits are arranged oblique to a central axis of the implantable body extending from the first end to the second end.

3. The deployable bone anchor of claim 1, wherein the living hinge portion comprises:
a first portion of the sidewall proximate the first end;
a first flex feature connecting the first portion to the first end;
a second portion of the sidewall proximate the second end;
a second flex feature connecting the second portion to the second end; and
a central component connecting the first portion and the second portion, wherein the central component comprises a third flex feature.

4. The deployable bone anchor of claim 3, wherein:
the first and second flex features have a first radius of curvature and the third flex feature has a second radius of curvature, wherein the second radius of curvature is greater than the first radius of curvature; and
the first and second flex features have a first thickness and the third flex feature has a second thickness, wherein the second thickness is greater than the first thickness.

5. The deployable bone anchor of claim 3, wherein:
the central component comprises a third portion of the sidewall connected to the first portion, a fourth portion of the sidewall connected to the second portion and a third flex feature connecting the third portion and the fourth portion; and
the third flex feature faces in an opposite radial direction as the first flex feature and the second flex feature.

6. The deployable bone anchor of claim 3, wherein:
the central component is located axially asymmetrically between the first end and the second end; or
the first portion of the sidewall and the second portion of the sidewall have different lengths.

7. The deployable bone anchor of claim 1, wherein:
the second attachment member (807) comprises a flange (816);
the third attachment member (808) comprises a threaded bore (822); and
the first attachment member (806) comprises a frustoconical socket (814).

8. The deployable bone anchor of claim 1, further comprising a prosthetic articulating component attachable to the first attachment member, the prosthetic articulating component configured to engage another component or bone in a sliding manner.

9. The deployable bone anchor of claim 1, wherein:
the sidewall comprises a plurality of living hinge portions spaced axially along the sidewall; and
the living hinge portion comprises a plurality of circumferential sections having different radial expansion configurations.

10. The deployable bone anchor of claim 1, further comprising a stop feature configured to limit movement of the first end and the second end toward each other via flexure of the living hinge portion, wherein the stop feature comprises first and second axially engageable features connected to the first end and the second end, respectively or angled surfaces of the living hinge portion that limit flexure of the living hinge portion.

11. A system for attaching an orthopedic implant to bone, the system comprising:
a deployment device (400) comprising:
a sleeve (402) having an internal channel extending along an axis; and
a drive shaft (404) extending through the sleeve; and
the deployable bone anchor of claim 1.

12. The system of claim 11, wherein:
the sleeve further comprises a handle (408); and
the drive shaft further comprises a knob (412).

13. The system of claim 11, wherein:
the sleeve comprises a distal tip having a tooth (416A, 416B) configured to engage the second attachment member (807); and
the sleeve comprises flexible arm (418A, 418B) to which the tooth (416A, 416B) is connected.

14. The system of claim 11, wherein:
the second attachment member comprises a threaded flange;
the drive shaft (404) comprises a threaded distal tip (414); and
the third attachment member (808) comprises a threaded bore (822).

15. The system of claim 11, wherein:
the first end includes a first cylindrical wall forming the first attachment member (806) and the second end includes a second cylindrical wall forming the third attachment member (808); and
the first cylindrical wall and the second cylindrical wall are configured to axially engage to prevent the drive shaft (404) from moving relative to the sleeve (402).

## Patentansprüche

1. Entfaltbarer Knochenanker für ein orthopädisches Implantat, wobei der entfaltbare Knochenanker Folgendes umfasst:
einen implantierbaren Körper (154), umfassend:
ein erstes Ende, umfassend:
ein erstes Anbringungselement (806), um Koppeln einer prothetischen Komponente an den implantierbaren Körper zu erleichtern; und
ein zweites Anbringungselement (807), um Koppeln einer Entfaltungsvorrichtung an den implantierbaren Körper zu erleichtern;
ein zweites Ende zur Einführung in einen Knochen, wobei das zweite Ende ein drittes Anbringungselement (808) umfasst, um Koppeln an die Entfaltungsvorrichtung zu erleichtern; und
eine Seitenwand (154, 202, 302, 702, 804, 852), die das erste Ende und das zweite Ende verbindet, wobei die Seitenwand Folgendes umfasst:
einen lebenden Scharnierabschnitt, der dazu konfiguriert ist, der Seitenwand zu ermöglichen, sich zu biegen, wenn das zweite Anbringungselement (807) und das dritte Anbringungselement (808) durch die Entfaltungsvorrichtung zueinander gezogen werden, wenn die Entfaltungsvorrichtung an dem implantierbaren Körper angebracht ist;
wobei die Seitenwand (154, 202, 302, 702, 804, 852) dazu konfiguriert ist, sich von einer erstreckten Konfiguration, in der sich die Seitenwand axial erstreckt, zu einer zusammengeklappten Konfiguration, in der die Seitenwand radial nach außen gebogen ist, zu entfalten; und
wobei der lebende Scharnierabschnitt dazu konfiguriert ist, Krümmung an der Seitenwand in der zusammengeklappten Konfiguration zu induzieren.

2. Entfaltbarer Knochenanker nach Anspruch 1, wobei:
die Seitenwand (154, 202, 302, 702, 804, 852) eine Vielzahl von Schlitzen (116A, 116B, 186A, 186B, 186C) umfasst, die sich durch die Seitenwand erstreckt; und
die Vielzahl von Schlitzen schräg zu einer zentralen Achse des implantierbaren Körpers angeordnet ist, die sich von dem ersten Ende zu dem zweiten Ende erstreckt.

3. Entfaltbarer Knochenanker nach Anspruch 1, wobei der lebende Scharnierabschnitt Folgendes umfasst:
einen ersten Abschnitt der Seitenwand nahe dem ersten Ende;
ein erstes Biegemerkmal, das den ersten Abschnitt mit dem ersten Ende verbindet;
einen zweiten Abschnitt der Seitenwand nahe dem zweiten Ende;
ein zweites Biegemerkmal, das den zweiten Abschnitt mit dem zweiten Ende verbindet; und
eine zentrale Komponente, die den ersten Abschnitt und den zweiten Abschnitt verbindet, wobei die zentrale Komponente ein drittes Biegemerkmal umfasst.

4. Entfaltbarer Knochenanker nach Anspruch 3, wobei:
das erste und das zweite Biegemerkmal einen ersten Krümmungsradius aufweisen und das dritte Biegemerkmal einen zweiten Krümmungsradius aufweist, wobei der zweite Krümmungsradius größer als der erste Krümmungsradius ist; und
das erste und das zweite Biegemerkmal eine erste Dicke aufweisen und das dritte Biegemerkmal eine zweite Dicke aufweist, wobei die zweite Dicke größer als die erste Dicke ist.

5. Entfaltbarer Knochenanker nach Anspruch 3, wobei:
die zentrale Komponente einen dritten Abschnitt der Seitenwand, der mit dem ersten Abschnitt verbunden ist, einen vierten Abschnitt der Seitenwand, der mit dem zweiten Abschnitt verbunden ist, und ein drittes Biegemerkmal, das den dritten Abschnitt und den vierten Abschnitt verbindet, umfasst; und
das dritte Biegemerkmal in eine entgegengesetzte radiale Richtung wie das erste Biegemerkmal und das zweite Biegemerkmal weist.

6. Entfaltbarer Knochenanker nach Anspruch 3, wobei:
sich die zentrale Komponente axial asymmetrisch zwischen dem ersten Ende und dem zweiten Ende befindet; oder
der erste Abschnitt der Seitenwand und der zweite Abschnitt der Seitenwand unterschiedliche Längen aufweisen.

7. Entfaltbarer Knochenanker nach Anspruch 1, wobei:
das zweite Anbringungselement (807) einen Flansch (816) umfasst;
das dritte Anbringungselement (808) eine Gewindebohrung (822) umfasst; und
das erste Anbringungselement (806) eine kegelstumpfförmige Fassung (814) umfasst.

8. Entfaltbarer Knochenanker nach Anspruch 1, ferner umfassend eine prothetische Gelenkkomponente, die an dem ersten Anbringungselement anbringbar ist, wobei die prothetische Gelenkkomponente dazu konfiguriert ist, eine andere Komponente oder einen anderen Knochen auf eine gleitende Weise in Eingriff zu nehmen.

9. Entfaltbarer Knochenanker nach Anspruch 1, wobei:
die Seitenwand eine Vielzahl von lebenden Scharnierabschnitten umfasst, die axial entlang der Seitenwand beabstandet sind; und
der lebende Scharnierabschnitt eine Vielzahl von Umfangsanteilen umfasst, die unterschiedliche radiale Ausdehnungskonfigurationen aufweisen.

10. Entfaltbarer Knochenanker nach Anspruch 1, ferner umfassend ein Anschlagmerkmal, das dazu konfiguriert ist, Bewegung des ersten Endes und des zweiten Endes zueinander über Biegung des lebenden Scharnierabschnittes zu begrenzen, wobei das Anschlagmerkmal ein erstes und ein zweites axial in Eingriff bringbares Merkmal, die jeweils mit dem ersten Ende und dem zweiten Ende verbunden sind, oder gewinkelte Flächen des lebenden Scharnierabschnittes, die Biegung des lebenden Scharnierabschnittes begrenzen, umfasst.

11. System zum Anbringen eines orthopädischen Implantats an Knochen, wobei das System Folgendes umfasst:
eine Entfaltungsvorrichtung (400), umfassend:
eine Hülse (402), die einen inneren Kanal aufweist, der sich entlang einer Achse erstreckt; und
eine Antriebswelle (404), die sich durch die Hülse erstreckt; und
den entfaltbaren Knochenanker nach Anspruch 1.

12. System nach Anspruch 11, wobei:
die Hülse ferner einen Griff (408) umfasst; und
die Antriebswelle ferner einen Knopf (412) umfasst.

13. System nach Anspruch 11, wobei:
die Hülse eine distale Spitze mit einem Zahn (416A, 416B) umfasst, die dazu konfiguriert ist, das zweite Anbringungselement (807) in Eingriff zu nehmen; und
die Hülse einen flexiblen Arm (418A, 418B) umfasst, mit dem der Zahn (416A, 416B) verbunden ist.

14. System nach Anspruch 11, wobei:
das zweite Anbringungselement einen Gewindeflansch umfasst;
die Antriebswelle (404) eine distale Gewindespitze (414) umfasst; und
das dritte Anbringungselement (808) eine Gewindebohrung (822) umfasst.

15. System nach Anspruch 11, wobei:
das erste Ende eine erste zylindrische Wand beinhaltet, die das erste Anbringungselement (806) bildet, und das zweite Ende eine zweite zylindrische Wand beinhaltet, die das dritte Anbringungselement (808) bildet; und
die erste zylindrische Wand und die zweite zylindrische Wand dazu konfiguriert sind, axial in Eingriff zu treten, um zu verhindern, dass sich die Antriebswelle (404) relativ zu der Hülse (402) bewegt.

## Revendications

1. Ancrage osseux déployable pour un implant orthopédique, l'ancrage osseux déployable comprenant :
un corps implantable (154) comprenant :
une première extrémité comprenant :
un premier élément de fixation (806) destiné à faciliter le couplage d'un composant prothétique au corps implantable ; et
un deuxième élément de fixation (807) destiné à faciliter le couplage d'un dispositif de déploiement au corps implantable ;
une seconde extrémité permettant l'insertion dans un os, la seconde extrémité comprenant un troisième élément de fixation (808) destiné à faciliter le couplage au dispositif de déploiement ; et
une paroi latérale (154, 202, 302, 702, 804, 852) reliant la première extrémité et la seconde extrémité, la paroi latérale comprenant :
une partie charnière mobile conçue pour permettre à la paroi latérale de fléchir lorsque le deuxième élément de fixation (807) et le troisième élément de fixation (808) sont tirés l'un vers l'autre par le dispositif de déploiement lorsque le dispositif de déploiement est fixé au corps implantable ;
ladite paroi latérale (154, 202, 302, 702, 804, 852) étant conçue pour se déployer à partir d'une configuration étendue dans laquelle la paroi latérale s'étend axialement vers une configuration repliée dans laquelle la paroi latérale est arquée radialement vers l'extérieur ; et
ladite partie charnière mobile étant conçue pour induire la courbure de la paroi latérale dans la configuration repliée.

2. Ancrage osseux déployable selon la revendication 1 :
ladite paroi latérale (154, 202, 302, 702, 804, 852) comprenant une pluralité de fentes (116A, 116B, 186A, 186B, 186C) s'étendant à travers la paroi latérale ; et
ladite pluralité de fentes étant agencée de manière oblique par rapport à un axe central du corps implantable s'étendant de la première extrémité à la seconde extrémité.

3. Ancrage osseux déployable selon la revendication 1, ladite partie charnière mobile comprenant :
une première partie de la paroi latérale à proximité de la première extrémité ;
un premier élément de flexion reliant la première partie à la première extrémité ;
une deuxième partie de la paroi latérale à proximité de la seconde extrémité ;
un deuxième élément de flexion reliant la deuxième partie à la seconde extrémité ; et
un composant central reliant la première partie et la deuxième partie, ledit composant central comprenant un troisième élément de flexion.

4. Ancrage osseux déployable selon la revendication 3 :
lesdits première et deuxième éléments de flexion présentant un premier rayon de courbure et ledit troisième élément de flexion présentant un second rayon de courbure, ledit second rayon de courbure étant supérieur au premier rayon de courbure ; et
lesdits premier et deuxième éléments de flexion présentant une première épaisseur et ledit troisième élément de flexion présentant une seconde épaisseur, ladite seconde épaisseur étant supérieure à la première épaisseur.

5. Ancrage osseux déployable selon la revendication 3 :
ledit composant central comprenant une troisième partie de la paroi latérale reliée à la première partie, une quatrième partie de la paroi latérale reliée à la deuxième partie et un troisième élément de flexion reliant la troisième partie et la quatrième partie ; et
ledit troisième élément de flexion étant orienté suivant une direction radiale opposée à celle du première élément de flexion et du deuxième élément de flexion.

6. Ancrage osseux déployable selon la revendication 3 :
ledit composant central étant situé axialement asymétriquement entre la première extrémité et la seconde extrémité ; ou
ladite première partie de la paroi latérale et ladite deuxième partie de la paroi latérale présentant des longueurs différentes.

7. Ancrage osseux déployable selon la revendication 1 :
ledit deuxième élément de fixation (807) comprenant une bride (816) ;
ledit troisième élément de fixation (808) comprenant un alésage fileté (822) ; et
ledit premier élément de fixation (806) comprenant une douille tronconique (814).

8. Ancrage osseux déployable selon la revendication 1, comprenant en outre un composant d'articulation prothétique pouvant être fixé au premier élément de fixation, le composant d'articulation prothétique étant conçu pour se mettre en prise avec un autre composant ou os de manière coulissante.

9. Ancrage osseux déployable selon la revendication 1 :
ladite paroi latérale comprenant une pluralité de parties charnière mobile espacées axialement le long de la paroi latérale ; et
ladite partie charnière mobile comprenant une pluralité de sections circonférentielles présentant différentes configurations d'expansion radiale.

10. Ancrage osseux déployable selon la revendication 1, comprenant en outre un élément de butée conçue pour limiter le mouvement de la première extrémité et de la seconde extrémité l'une vers l'autre par flexion de la partie charnière mobile, ledit élément de butée comprenant des premier et deuxième éléments pouvant se mettre en prise axialement reliés à la première extrémité et à la seconde extrémité, respectivement ou des surfaces angulaires de la partie charnière mobile qui limitent la flexion de la partie charnière mobile.

11. Système destiné à fixer un implant orthopédique à un os, le système comprenant :
un dispositif de déploiement (400) comprenant :
un manchon (402) comportant un canal interne s'étendant le long d'un axe ; et
un arbre d'entraînement (404) s'étendant à travers le manchon ; et
l'ancrage osseux déployable selon la revendication 1.

12. Système selon la revendication 11 :
ledit manchon comprenant en outre une poignée (408) ; et
ledit arbre d'entraînement comprenant en outre une molette (412).

13. Système selon la revendication 11 :
ledit manchon comprenant une pointe distale comportant une dent (416A, 416B) conçue pour se mettre en prise avec le deuxième élément de fixation (807) ; et
ledit manchon comprenant un bras flexible (418A, 418B) auquel la dent (416A, 416B) est reliée.

14. Système selon la revendication 11 :
ledit deuxième élément de fixation comprenant une bride filetée ;
ledit arbre d'entraînement (404) comprenant une pointe distale filetée (414) ; et
ledit troisième élément de fixation (808) comprenant un alésage fileté (822).

15. Système selon la revendication 11 :
ladite première extrémité comprenant une première paroi cylindrique formant le premier élément de fixation (806) et ladite seconde extrémité comprenant une seconde paroi cylindrique formant le troisième élément de fixation (808) ; et
ladite première paroi cylindrique et ladite seconde paroi cylindrique étant conçues pour se mettre en prise axialement de manière à empêcher le déplacement de l'arbre d'entraînement (404) par rapport au manchon (402).
